# EUROPEAN PATENT APPLICATION

(11) **EP 3 081 191 A1**
(43) Date of publication of application: **19.10.2016**
(21) Application number: 16275024.4
(22) Date of filing: 15.02.2016
(51) Int. Cl.: A61F 2/01, A61B 17/12

(54) **MEDICAL DEVICE WITH CONNECTED LEGS**

(30) Priority: 13.04.2015 GB 201506221
(71) Applicant: Cook Medical Technologies LLC, Bloomington, IN 47404 (US)
(72) Inventor: Johnsen, Jeppe, 5871 Froerup (DK); Klarskov, Lisbeth, 4320 Lejre (DK)
(74) Representative: Williams Powell

(57) **Abstract**

A vascular filter (30) includes a hub (32) and a basket portion (38). The filter (30) is formed from a plurality of strands (10) of a biocompatible material such as a polymer or a super-elastic metal alloy. The strands (10) are intertwined to form the hub (32) the strands are then secured by forming a knot such as a crown knot (20) or a splice (60), and the free ends of the strands 10 form a basket portion (38) of the filter (30). The filter (30) may thus be formed only by the strands (10), thereby avoiding problems associated with known methods of joining legs to form a filter.

## Description

The present invention relates to a medical device, in particular to a medical device having connected legs, and a method of making such a medical device.

Filtering devices that are percutaneously placed in the vena cava have been available for a number of years. A need for filtering devices arises in trauma patients, orthopaedic surgery patients, neurosurgery patients, or in patients having medical conditions requiring bed rest or non-movement because of the likelihood of thrombosis in the peripheral vasculature of patients. The thrombi may break away from the vessel wall, and, depending on the size of the thrombi, pose a serious risk of pulmonary embolism when blood clots migrate from the peripheral vasculature through the heart and into the lungs.

Filter devices usually comprise a plurality of legs attached to a hub. The legs expand after placement within a body lumen so that the proximal ends of the legs engage with a vessel wall. Known methods of joining the legs to the hub include crimping, welding and soldering.

Examples of known medical devices are disclosed in WO 2010/015397, WO 2011/032720, US 6,652,571, US 5,382,259, US 2002/0169498, US 2002/0052644, US 2004/0054396, US 5,545,211, US 6,221,100, US 6,241,757, US 6,007,574, US 2003/0149475, US 2002/0004667, US 2003/0130724, US 2005/0256563, US 2006/0195175, and US 2010/0228281.

The present invention seeks to provide an improved medical device for implantation in a body lumen.

According to an aspect of the present invention, there is provided medical device for implantation in a body lumen, the medical device including a hub and a plurality of legs extending from the hub, the legs being connected to one another by being intertwined with one another along a portion of a length thereof at the hub, the legs thereby being supported by one another.

Fixing the legs to one another by intertwining them can avoid problems such as undesirable material deformation, annealing, contamination, and material phase transitions that may occur with known joining methods.

In an embodiment the legs are secured to one another by means of a knot. The knot may be a splice, a crown knot or a whipping knot, for example.

In an embodiment the medical device includes two pluralities of legs and the two pluralities of legs are secured to one another using a splice at the hub.

The knot may be formed only by the legs.

The medical device may include a basket portion, the basket portion including free ends of the legs, the free ends of the legs extending outwardly from the hub, and being expandable to engage walls of the body lumen.

The device may be a filter or an occlusion device.

The intertwined portion of the legs may form the hub.

The legs may be intertwined by being twisted together or by being knitted together.

The legs may be polymeric or metallic for example. In an embodiment the legs are a super-elastic metal alloy.

According to another aspect of the present invention, there is provided a method of making a medical device, the medical device including a hub and a plurality of legs extending from the hub, including: connecting the legs to one another by intertwining the plurality of legs along a portion of a length thereof at the hub, the legs thereby supporting one another.

The method may include heat-setting the device into a desired shape.

In an embodiment, the method includes securing the legs together by means of a knot. The knot may be a splice, a crown knot or a whipping knot, for example.

The method may include intertwining the legs of two pluralities of legs and securing these together at the hub using a splice.

The knot may be formed only by the legs.

The method may include forming a basket portion from free ends of the legs, the free ends of the legs extending outwardly from the hub, and being expandable to engage walls of the body lumen.

The device may be a filter or an occlusion device.

The hub may be formed by intertwining the legs.

The method may include intertwining the legs by twisting them together or by knitting them together.

The legs may be polymeric or metallic for example. In an embodiment the legs are a super-elastic metal alloy.

According to another aspect of the present invention, there is provided a method of deploying a medical device as specified above in a blood vessel, including: introducing the medical device to a treatment site within a patient's body, and allowing the medical device to expand.

An embodiment of the present invention is described below, by way of example only, with reference to the accompanying drawings, in which:
Figure 1 illustrates the forming of a crown knot using three intertwined strands;
Figure 2 illustrates the formed crown knot after tightening;
Figure 3 illustrates an embodiment of a vascular filter including a crown knot;
Figure 4 schematically illustrates the vascular filter of Figure 3 after deployment in the inferior vena cava;
Figures 5 to 7 illustrate the forming of a splice between two three-stranded hubs;
Figures 8 and 9 illustrate a vascular filter including a splice; and
Figure 10 schematically illustrates the vascular filter of Figures 8 and 9 after deployment in the inferior vena cava.

It is to be understood that the drawings are schematic only and do not show the elements in proportion. In many instances, the Figures show scaled up components to assist the reader. The skilled person will readily appreciate the typical dimensions and proportions of the various elements depicted and will also know that these will also vary in dependence upon the nature of the vessel in which the device is to be implanted.

In this description, the term distal, when used with respect to the medical device or a component thereof, denotes an end that it downstream with respect to blood flow. The term proximal is used to denote an end that is upstream with respect to blood flow. However, when referring to an elongated device such as a sheath or guide wire, the term distal is used to refer to an end of a component which in use is furthest from the surgeon during the medical procedure, including within a patient. The term proximal is used to refer to an end of a component closest to the surgeon and in practice in or adjacent an external manipulation part of the device.

Known medical devices are often formed by joining a plurality of struts, usually using methods such as crimping, welding or soldering. Such methods can result in undesirable material deformation, annealing or contamination.

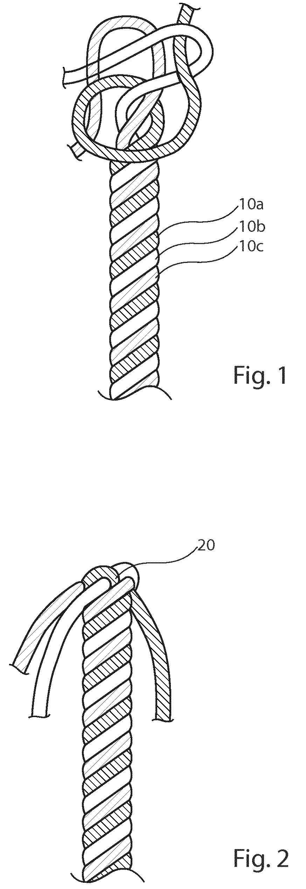

Figures 1 to 3 illustrate the formation of medical device for implantation in a body lumen. In this example, the medical device is a vascular filter 30 formed from three intertwined strands 10 of, in this example, biocompatible, self-expanding polymeric material, such as polyactic acid (PLA), polyglycolic acid (PGA), or derivatives of these. In other embodiments the strands 10 may be metallic. For example, they may be formed from a super-elastic metal alloy such as Nitinol.

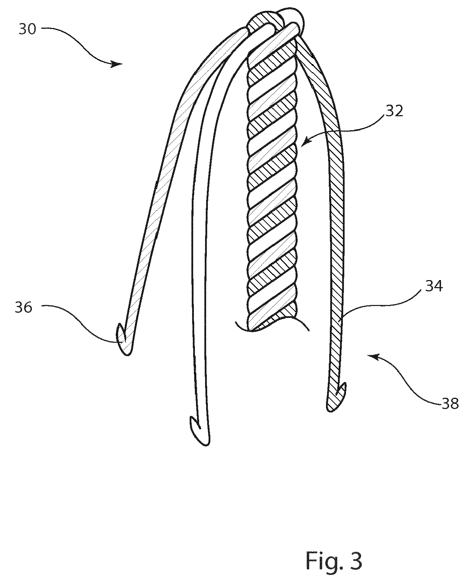

As shown in Figure 3, the filter 30 includes a hub 32, which, in this embodiment, is formed by the intertwined strands 10. The hub preferably has a diameter of below 3mm. The filter 30 includes a basket portion 38. The three strands 10 exit the hub 32 at its distal end and extend alongside and beyond the hub 20 in a proximal direction to form the basket portion 38 from the legs 34 of the filter 30. The basket portion 38 includes the free ends of the legs 34, which, in use, expand and engage the walls of a body lumen such as a blood vessel. The legs 34 may be provided with barbs 36 to assist in anchoring the filter 30 within the lumen. The overall length of the device is preferably between 30mm and 100mm and in a preferred embodiment, the filter functions in vessels having a diameter in the range of at least 15 to 30mm.

The filter 30 is formed by twisting together the strands 10 to form the hub 32 of the vascular filter 30. To secure the strands 10, a knot is formed at the distal end of the hub 32. Preferably the knot is formed only by the strands 10. In this embodiment a crown knot 20 is used. A crown knot is the first step in the formation of a back splice, which is usually used in a method of preventing the end of a rope from fraying.

The crown knot 20 may be formed by spreading out the three strands 10. A first strand 10a is looped in the same direction as the twist and so as to overlie a second strand 10b. The second strand 10b is then looped in the same direction as the twist so as to overlie the first strand 10a and a third strand 10c. The third strand 10c is then looped in the same direction as the twist so as to overlie the second strand 10b. The third strand 10c is then tucked through the loop formed by the first strand 10a. The crown knot 20 can then be tightened by pulling the strands 10 so that it sits snugly at the distal end of the hub 32.

The strands 10 at the proximal end of the hub 32 may be secured to one another by any suitable means. They could be glued, or sewn together using a soft thread, or tied or spliced in a similar manner to the distal end.

Where necessary, for example, depending on the material from which the filter 30 is formed, the device is heat set. For example, if the device is made of Nitinol, the device is heat set in a known manner to exploit its super-elastic properties.

The legs 34 of the filter 30 are thus connected to one another and supported by one another by being intertwined with one another. Furthermore, the legs 34 are secured to one another by means of the knot (crown knot 20).

In practice, the filter 30, or other medical device, will be deployed endoluminally to the treatment site, typically through a catheter or sheath, with the legs 34 radially compressed in the catheter and only able to expand radially outwardly once released from the introducer assembly, in a manner well known in the art. The expansion force when measured on the contact points on the legs of the filter should be 0.05N to 0.5N.

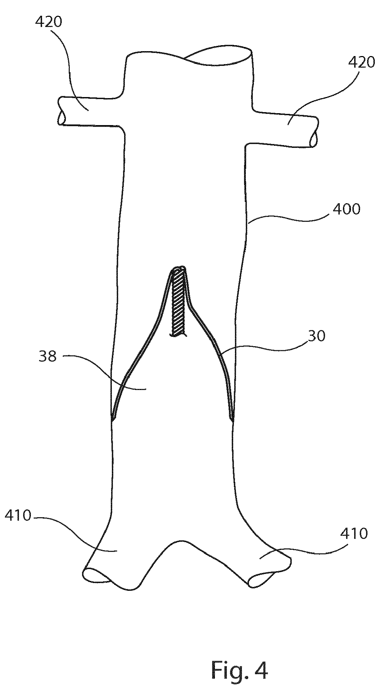

Figure 4 is a schematic illustration of the vascular filter 30 after deployment within a blood vessel, in this case, the inferior vena cava 400. The vascular filter 30 is located between the common iliac veins 410 and the renal veins 420. Blood flows into the basket portion 38 where any thrombus in the blood flow may be trapped and prevented from entering the heart and lungs.

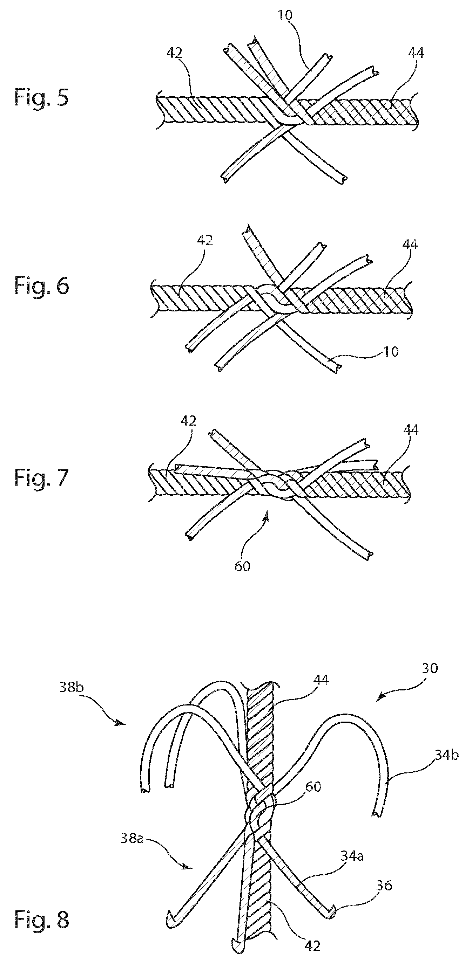

Another embodiment of medical device for implantation in a body lumen, in this example also a vascular filter 30, is illustrated in Figures 5 to 9. This embodiment of filter 30 includes six legs 34, and is formed by joining two pluralities of strands 10 of biocompatible material (for example a polymer or metal alloy) together at a hub 32. In this embodiment, the knot is a splice 60. It is preferred that the knot is formed only by the strands 10. A first hub portion 42, formed from three strands 10 twisted together, is joined with a separate second hub portion 44 of three strands 10 twisted together by means of the splice 60. As shown, in particular, in Figure 8, the vascular filter 30 includes a hub 32 formed from the first and second hub portions 42,44, joined by the splice 60 at a substantially central hub portion.

Extending from the substantially central hub portion are, in this embodiment, six legs 34, three proximal legs 34a and three distal legs 34b. The filter 30 includes first and second basket portions 38a, 38b, including the free ends of the proximal legs 34a and the distal legs 34b respectively. The three proximal legs 34a extend directly out of the splice 60 in a proximal direction at an angle to the hub 32. The legs 34a include barbs 36 at their proximal ends to assist in anchoring the filter 30 within a body lumen such as a blood vessel. The barbs 36 could be formed by plastic deformation of the ends of the strands 10 or by heat-setting where the strands 10 are formed from Nitinol. In some embodiments pre-formed barbs 36 could be attached to the strands 10, for example using glue, laser welding or stitching. The three distal legs 34b extend directly out of the splice 60 in a distal direction at an angle to the hub 32. The three distal legs 34b then, in this embodiment, curve to extend in a proximal direction. The distal legs 34b may engage the wall of the body lumen and assist in correctly aligning the filter 30. The distal legs 34b preferably do not include barbs.

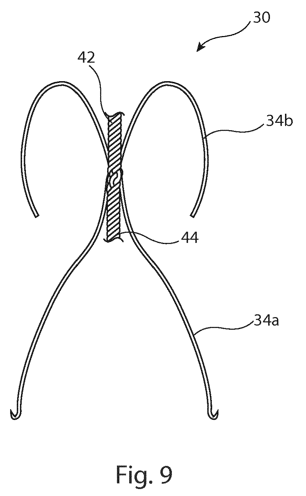

Figure 9 schematically illustrates the overall shape of the filter of Figure 8 (not all legs are shown).

As indicated above, the splice 60 joins two hub portions 42,44 each formed from three strands 10 twisted together along a portion of their lengths. A portion of each strand 10 of sufficient length to form the splice 60 and the legs 34 is left untwisted. The untwisted leg portions of the strands 10 are brought together and interleaved. Each untwisted leg portion is then, in turn, tucked under a twist of the opposing hub portion 42,44 three to five times to join the two hub portions 42,44 securely as illustrated in Figures 5 to 7.

The splice 60 should be sufficient to secure the strands 10 in the hub 42,44. However, if necessary, the strands 10 at the ends of the hub portions 42, 44 could be glued or sewn together using a soft thread.

Where necessary, for example, depending on the material from which the filter 30 formed, the device is heat set.

The legs 34 of the filter 30 are thus connected to one another and supported by one another by being intertwined with one another. Furthermore, the legs 34 are secured to one another by means of the knot (splice 60).

The diameters given above for the embodiment illustrated in Figure 3 apply equally to this embodiment.

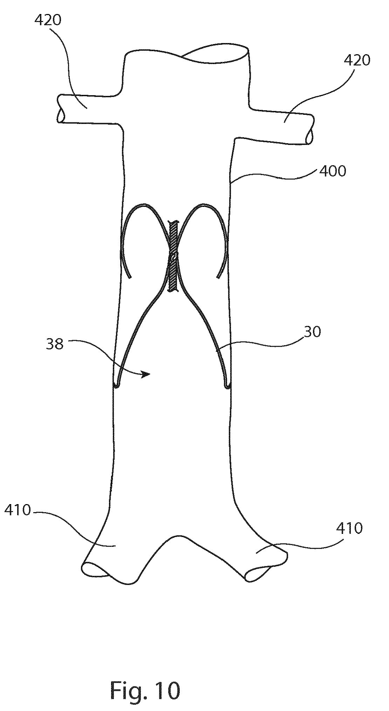

The vascular filter 30 of Figures 8 and 9 would be deployed in a manner analogous to that described above for the embodiment of Figure 3. Figure 10 schematically illustrates the vascular filter 30 of Figures 8 and 9 after deployment within the inferior vena cava. The description above for Figure 4 applies equally here.

It will be appreciated by the skilled person that the embodiment illustrated in Figures 5 to 9 could readily be adapted to produce a medical device having an overall substantially hourglass shape or double-cone shape. This could be achieved by allowing the distal legs 34b to continue to extend in the distal direction. In other words, the device may include a distally extending basket as well as a proximally extending basket.

In the illustrated embodiments the strands 10 are wires having a generally circular cross-section. Other types of strand are also possible. For example, the strands could be ribbon-like, having a rectangular cross-section.

Generally, all of the strands 10 are formed from the same material. However in some embodiments it may be desirable to use strands of different material within the same device. A second material could function as an adhesive. For example, using steel wire and intertwining a soft polymer could lock the construction.

The embodiments illustrated in the Figures show the strands 10 simply twisted together. In modifications, the strands 10 could be intertwined using other methods. For example, they could be plaited, or knitted together.

The number of legs 34 shown in the illustrated embodiments is exemplary only. The skilled person would appreciate that filter devices 30 with a greater number of legs 34 could be made by intertwining a greater number of strands 10. The number of legs 34 may, for example, be 6 to 16, for example 8. The above teachings apply equally to such devices. In particular, it is to be noted that increasing the number of strands does not necessarily render the intertwining and knotting more complex. The strands 10 could be gathered together into, for example, three groups for the purposes of intertwining and knotting, and only being separated into separate legs 34 as they exit the hub 32; 42, 44.

The described techniques are not only useful for filter devices; they could be applied to other types of medical device. For example, graft material could be attached to the legs 34 to form an occlusion device.

Use of the methods disclosed herein to connect legs of a medical device can avoid undesirable deformations and material phase transitions, annealing and contamination that may occur with prior art methods such as crimping, welding or soldering. The presently disclosed methods may avoid fatigue failure and may lower process costs. However, in some embodiments it may be desirable to include some bonding, welding or crimping, for example, to provide additional security to the intertwining.

What has been described and illustrated herein is a preferred embodiment of the invention along with some of its variations. The terms, descriptions and Figures used herein are set forth by way of illustration only and are not meant as limitations. Those skilled in the art will recognise that many variations are possible within the spirit and scope of the invention, which is intended to be defined by the following claims, and their equivalents, in which all terms are meant in their broadest reasonable sense unless otherwise indicated.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in GB 1506661.9, from which this application claims priority, and in the abstract accompanying this application are incorporated herein by reference.

## Claims

1. A medical device (30) for implantation in a body lumen (400), the medical device including a hub (32; 42,44) and a plurality of legs (34) extending from the hub, the legs being connected to one another by being intertwined with one another along a portion of a length thereof at the hub, the legs thereby being supported by one another.

2. A method of making a medical device (30), the medical device including a hub (32; 42,44) and a plurality of legs (34) extending from the hub, including: connecting the legs to one another by intertwining the plurality of legs along a portion of a length thereof at the hub, the legs thereby supporting one another.

3. A method as claimed in claim 2, including heat-setting the device (30) into a desired shape.

4. A medical device (30) as claimed in claim 1 or a method as claimed in claim 2 or 3, wherein the legs (34) are secured to one another by means of a knot (20; 60).

5. A medical device (30) or a method as claimed in claim 4, wherein the knot is a splice (60).

6. A medical device (30) as claimed in claim 1, 4 or 5 or a method as claimed in any of claims 2 to 5, including two pluralities of legs and the two pluralities of legs are secured to one another using a splice (32) at the hub (42,44).

7. A medical device (30) or a method as claimed in claim 4, wherein the knot is a crown knot (20) or a whipping knot.

8. A medical device (30) as claimed in claim 1 or any of claims 4 to 7 or a method as claimed in any of claims 2 to 7, wherein the knot (20; 60) is formed only by the legs (34).

9. A medical device (30) as claimed in claim 1 or any of claims 4 to 8 or a method as claimed in any of claims 2 to 8, wherein the medical device includes a basket portion (38), the basket portion including free ends of the legs (34), the free ends of the legs extending outwardly from the hub (32; 42,44) in a proximal direction, and being expandable to engage walls of the body lumen.

10. A medical device (30) as claimed in claim 1 or any of claims 4 to 9 or a method as claimed in any of claims 2 to 9, wherein the device is a filter or an occlusion device.

11. A medical device (30) as claimed in claim 1 or any of claims 4 to 10 or a method as claimed in any of claims 2 to 10, wherein the intertwined portion of the legs (34) forms the hub (32; 42,44).

12. A medical device (30) as claimed in claim 1 or any of claims 4 to 11 or a method as claimed in any of claims 2 to 11, wherein the legs (34) are intertwined by being twisted together.

13. A medical device (30) as claimed in claim 1 or any of claims 4 to 11 or a method as claimed in any of claims 2 to 11, wherein the legs (34) are intertwined by being knitted together.

14. A medical device (30) as claimed in claim 1 or any of claims 4 to 13 or a method as claimed in any of claims 2 to 13, wherein the legs (34) are polymeric.

15. A medical device (30) as claimed in claim 1 or any of claims 4 to 14, or a method as claimed in any of claims 2 to 14, wherein the legs (34) are metallic, optionally wherein the legs are a super-elastic metal alloy.
